**Europäisches Patentamt**

**European Patent Office**

**Office européen des brevets**

(19)

(11) Veröffentlichungsnummer : **0 478 499 A1**

(12) # EUROPÄISCHE PATENTANMELDUNG

(21) Anmeldenummer : **91810689.9**

(22) Anmeldetag : **29.08.91**

(51) Int. Cl.$^5$ : **A61M 1/10, F04B 43/08**

(30) Priorität : **26.09.90 CH 3095/90**

(43) Veröffentlichungstag der Anmeldung :
**01.04.92 Patentblatt 92/14**

(84) Benannte Vertragsstaaten :
**AT CH DE FR GB IT LI NL SE**

(71) Anmelder : **GEBRÜDER SULZER
AKTIENGESELLSCHAFT
Zürcherstrasse 9
CH-8401 Winterthur (CH)**

(72) Erfinder : **Marcel, Jufer, Prof. EPFL
6, Ch. d'Orgiuz
CH-1110 Morges (CH)**
Erfinder : **Matthias, Krause
Rue du Paradis
CH-1967 Bramois (CH)**
Erfinder : **Ruchet, Claude
Pl. Meuniére 33
CH-1950 Sion (CH)**

(54) **Intermittierend fördernde Pumpe.**

(57)    Die Erfindung zeigt eine intermittierend fördernde Pumpe zum Fördern von Blut, die aus einer Rückschlagklappe (2) durch die Blut zufliesst, und aus einem Pumpelement (1), das Blut gegen einen höheren Druck durch eine Rückschlagklappe (3) ausstösst, besteht, wobei das Pumpelement eine deformierbare Kammer (4) und einen Antrieb (6) mit einem auf die Kammer (4) wirkenden Stosskörper (5) aufweist. Die Kammer (4) besteht aus einem druckfesten deformierbaren Sack (9) während der Antrieb (6) aus einem elektrischen Hohlwellenmotor (10) besteht. Mit der Drehung von dessen Hohlwelle (11) wirkt ein über Mutter (13) und Spindel (14) gebildeter Linearantrieb auf den Stosskörper (5) ein, wobei Mutter (13) und Spindel (14) innerhalb des als Hohlwelle (11) ausgebildeten Rotors (12) des Elektromotors angeordnet sind. Bei Ausführung des Sacks (9) in torus-ähnlicher Form (9a) sind Verschiebeelemente (13, 14) des Linearantriebes darin einschiebbar.

EP 0 478 499 A1

Fig.3

Die Erfindung handelt von einer intermittierend fördernden Pumpe zum Fördern von Blut bestehend aus einer Rückschlagklappe, durch die Blut zufliesst, und aus einem Pumpelement, das Blut gegen einen höheren Druck durch eine Rückschlagklappe ausstösst, wobei das Pumpelement eine deformierbare Kammer und einen Antrieb mit einem auf die Kammer wirkenden Stosskörper aufweist.

Pumpsysteme zur Unterstützung der Herztätigkeit eines Patienten, der auf eine Herztransplantation wartet, werden von verschiedenen Institutionen und Herstellern vorgeschlagen.

Eine erste Gruppe ordnet den Antrieb an der Peripherie einer deformierbaren Kammer und nicht in der Deformationsrichtung der Kammer an:

– Die Entwicklung der Stanford University mit der Firma NOVACOR MEDICAL (Portner, P.M., P.E.Oyer, J.S.Jassawalla, H.Chen, P.J.Miller, D.H.LaForge, G.F.Green, N.E.Shumway: A Totally Implantable Ventricular Assist Device for End-Stage Heart Disease, aus: Unger, Felex: Assisted Circulation 2, Springer Verlag, Berlin, Heidelberg, New York, Tokyo 1984, Seiten 115-140) zeigt mit dem Typ NOVACOR MK 22 C zwei an einem Trägerkörper jeweils mit Scharnier schwenkbare Elektromagnete, die sich in einem kleinen Abstand gegenüberliegen. Mit dem Betätigen eines Magnets werden Biegefedern freigegeben die bis zur Deformationsachse der Kammer hineinragen und jeweils auf eine Druckplatte wirken, sodass die Kammer durch zwei Hubbewegungen zusammengepresst wird.

– Die Entwicklung vom Massachusetts General Hospital mit der Firma ABIOMED (Nosé, Y.: Totally Implantable Artificial Organ; Cardiac Prosthesis, Artificial Organs, Vol. 10, No. 2, 1986, Seiten 102-113) zeigt zwei Kammern in Serie, die durch Rückschlagklappe miteinander verbunden sind. Durch wechselndes Umpumpen einer hydraulischen Flüssigkeit mit einer Pumpe wird jeweils das Volumen der einen oder anderen Kammer verringert.

Eine zweite Gruppe ordnet den Antrieb in der Deformationsrichtung der Kammer an und deformiert die Kammer durch Verschieben einer Membrane gegen eine Gehäusewand:

– Die Entwicklung des Texas Heart Institute mit der Firma GOULD INC. (Altieri, Frank D.: Status of Implantable Energy Systems do Actuate and Control Ventricular Assist Devices, Artificial Organs, Vol. 7, No. 1, 1983, Seiten 5-20) zeigt eine durch eine Membran abgeschlossene Kammer. Ein kontinuierlich drehender Elektromotor dreht über ein Untersetzungsgetriebe eine Kurvenscheibe mit drei Hochpunkten, welche auf drei Hebel wirken um über eine Stossplatte die Membran zu verschieben.

– Die Entwicklung des Childrens Hospital Medical Center (Boston) mit der Firma Thermetics Inc. (Gernes, D.B., W.F.Bernard, W.C.Clay, C.W.Sherman, D.Burke: Development of an Implantable, Integrated, Electrically Powered Ventricular Assist System, Vol. XXIX Transactions -American Society for Artificial Internal Organs, 1983, Seiten 546-550) zeigt mit dem Typ MK IV einen langsamen drehenden Elektromotor der pro Umgang zwei Führungsrollen auf einer Schraubenlinie aufwärts und auf einer Kurve wieder abwärts in die Ausgangslage bewegt, um eine Stossplatte gegen eine Membran zu bewegen.

– Die Entwicklung der Pennsylvania State University (Weiss, W.J.: in Möller, D.P.F., Bücherl, E.S.: Proceedings Second World Symposium Artificial Heart, Volume 1, Verlag: Friedrich Vieweg & Sohn, 1986, Seite 120) zeigt einen Gleichstrommotor, der eine schraubenförmige Kurve in einem Zylindermantel dreht, um die Bewegung über Rollen auf eine in der Zylinderachse verschiebbare Stossplatte zu übertragen, welche auf eine Membran einwirkt.

– Die Entwicklung der Cleveland Clinic Foundation mit der Firma NIMBUS INC. (Nosé, Y.: Totally Implantable Artificial Organ: Cardiac Prosthesis, Artificial Organs, Vol. 10,No. 2, 1986, Seiten 102-113) zeigt eine Mikropumpe die eine Trägerflüssigkeit hin- und herpumpt, welche mit Druck eine Kammer deformiert, damit das in derselben enthaltene Blut ausgestossen wird.

Pumpen dieser Art können mit dem Herzen eines Säugetiers oder Menschen z.B. zu der linken Herzkammer parallel oder in Serie angeschlossen werden, um die Herztätigkeit zu unterstützen. Falls so ein System als Implantat vorgesehen ist, sollte es bezüglich zulässigem Gewicht, zulässigem beanspruchten Volumen, Zuverlässigkeit und Lebensdauer sehr hohe Anforderungen erfüllen. Die hier zitierten Systeme sind in dieser Hinsicht nur begrenzt als Implantat geeignet.

Aus ethischen Ueberlegungen hat die Erfindung die Aufgabe, eine intermittierende Pumpe neben einem geschwächten natürlichen Herzen einzusetzen, um einen Teil von dessen Schwäche zu überbrücken, d.h. bei einem Versagen der mechanischen Pumpe wäre der Patient in der gleichen Situation wie vor deren Einsatz, nämlich auf sein natürliches Herz angewiesen. Es besteht die medizinische Erfahrung, dass blutpumpende Aggregate, die von ausserhalb des Körpers die Herztätigkeit unterstützen, mittelfristig Infektionen über die Durchgänge in der Körperhaut verursachen. Ausserdem ist der Aktionsradius eines Patienten, der über Zuleitungen, wie an einer Herz-Lungen-Maschine, hängt, auf wenige Meter beschränkt.

Hierzu bringt die Erfindung einen Beitrag. Aufgabe der Erfindung ist es, die konstruktiven Voraussetzungen für ein kompaktes und leichtes Pumpsystem zu schaffen, das in der Nähe des natürlichen Herzens implantierbar ist, und das den hohen Anforderungen besser genügt.

EP 0 478 499 A1

Gemäss der Erfindung wird die Aufgabe dadurch erfüllt, dass die Kammer aus einem druckfesten deformierbaren Sack besteht, dass der Antrieb aus einem elektrischen Hohlwellenmotor besteht, dass mit der Drehung dessen Hohlwelle ein über Mutter und Spindel gebildeter Linearantrieb auf den Stosskörper einwirkt und dass Mutter und Spindel innerhalb des als Hohlwelle ausgebildeten Rotors des Elektromotors angeordnet sind.

Die Vorteile der Erfindung sind darin zu sehen, dass ein für sich gekapseltes System implantierbar ist, dass Gewicht und Volumen des Implantats klein ausfallen, dass die bewegten Massen des Implantats gering sind, dass die rotierende Bewegung mit wenigen Teilen in eine Linearbewegung übertragen wird und dass alle bewegungsübertragende Elemente für geringe spezifische Belastungen und für hohe Lebensdauer ausgelegt sind. Die abhängigen Ansprüche 2 bis 12 beziehen sich auf vorteilhafte Weiterbildungen der Erfindung.

Im folgenden wird die Erfindung anhand von Ausführungsspielen beschrieben. Es zeigen:

Fig. 1 Eine schematische Skizze mit dem Bluttransport durch ein Pumpelement;

Fig. 2 ein schematisches Schnittbild einer erfindungsgemässen Pumpe mit einem Pumpelement, das als Kammer einen einfachen Sack aufweist;

Fig. 3 ein schematisches Schnittbild einer erfindungsgemässen Pumpe mit einem Pumpelement, das als Kammer einen torus-ähnlichen Sack aufweist;

Fig. 4 einen Ausschnitt aus Fig. 3 mit zusammengepresstem Sack;

Fig. 5 einen Ausschnitt gemäss Fig. 3 jedoch mit einer Kammer, die durch Membran und Gehäuse gebildet ist;

Fig. 6 einen Ausschnitt gemäss Fig. 5 mit nur einem Gehäusedeckel, der über die Membran zum Gehäuse dichtet; und

Fig. 7 ein schematisches, mittig unterbrochenes Schnittbild einer erfindungsgemässen Pumpe, die zwei wechselseitig deformierbare torus-ähnliche Säcke aufweist, wobei in der oberen Schnitthälfte annähernd die linke Endlage und in der unteren Schnitthälfte annähernd die rechte Endlage der Mutter gezeigt ist.

Die Figuren zeigen eine intermittierend fördernde Pumpe zum Fördern von Blut, die aus einer Rückschlagklappe durch die Blut zufliesst, und aus einem Pumpelement, das Blut gegen einen höheren Druck durch eine Rückschlagklappe ausstösst, besteht, wobei das Pumpelement eine deformierbare Kammer und einen Antrieb mit einem auf die Kammer wirkenden Stosskörper aufweist. Die Kammer besteht aus einem druckfesten deformierbaren Sack, während der Antrieb aus einem elektrischen Hohlwellenmotor besteht. Mit der Drehung von dessen Hohlwelle wirkt ein über Mutter und Spindel gebildeter Linearantrieb auf den Stosskörper ein, wobei Mutter und Spindel innerhalb des als Hohlwelle ausgebildeten Rotors des Elektromotors angeordnet sind. Bei Ausführung des Sacks in torus-ähnlicher Form sind Verschiebeelemente des Linearantriebes darin einschiebbar.

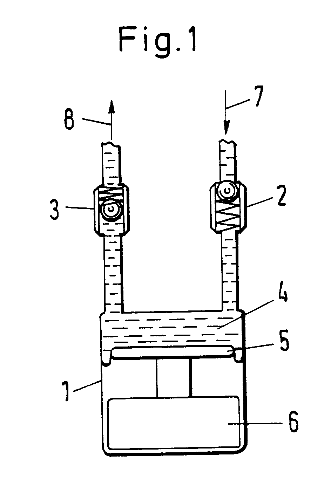

In Fig. 1 ist das Prinzip der Pumpe dargestellt. Blut strömt über eine Rückschlagklappe 2 oder ein Rückschlagventil zu einer Kammer 4, die durch Deformation in ihrem Volumen veränderlich ist. Ein Stosskörper 5, der von einem Antrieb 6 angetrieben wird, deformiert die Kammer 4 und stösst Blut über eine Rückschlagklappe oder - Ventil 3 aus. Mit der Rücklaufbewegung vom Stosskörper 5 strömt Blut über die Rückschlagklappe 2 nach, während Rückschlagklappe 3 geschlossen ist. Die Rückzugbewegung des Stosskörpers 5 erfolgt zwangsläufig, wobei der Stosskörper 5 sich von der Wand der Kammer 4 ablösen kann, damit kein Unterdruck in der Kammer 4 entsteht und damit der Druck des zuströmenden Blutes hauptsächlich zum Ausbeulen der deformierten Kammer 4 verwendet wird.

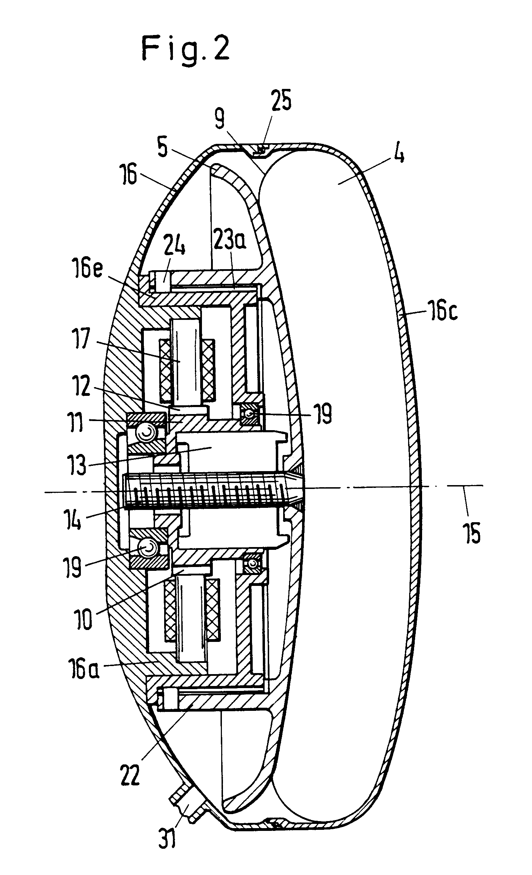

In Fig. 2 ist ein Schnitt durch ein diskus-ähnliches Pumpelement 1 mit einer Längsachse 15 dargestellt, die bis auf die nicht gezeigten Anschlussleitungen zwischen Kammer 4 und den Rückschlagklappen 2, 3 auch Symmetrieachse für die Anordnung ist. Das Gehäuse 16 besteht aus zwei hermetisch über eine statische Dichtung 25 verschlossene Hälften 16a, 16c und einer Zwischenwand 16e. Die Kammer 4 die durch einen druckfesten deformierbaren Sack 9 gebildet wird, stützt sich am Gehäuse 16c ab, an dem auch die Anschlussöffnungen zu den Rückschlagklappen 2, 3 angebracht sind. Durch eine Anschlussöffnung 31 kann ein Teil des im Gehäuse 16 befindlichen Gases beim Füllen der Kammer 4 in eine (hier nicht gezeigte) Ausgleichskammer geführt werden, um einen weichen Puffer zu erzeugen.

Antrieb 6 und Stosskörper 5 sind mit Abstützung auf den Gehäuseteilen 16a und 16e angeordnet. Das treibende Aggregat ist ein elektrischer Hohlwellenmotor 10 mit einem Statorteil 17, das im Gehäuseteil 16a befestigt ist, und mit einem Rotor 12, der über Kugellager 19 in den Gehäuseteilen 16a und 16e abgestützt ist. Der Rotor 12 besteht aus einer Hohlwelle 11 und nimmt eine Mutter 13 in sich auf, mit deren Drehung eine durch den Rotor 12 verlaufende Spindel 14 angetrieben wird. Die Spindel 14 ist mit dem Stosskörper 5 starr verbunden, der mit einem Kragen 22 am Gehäuseteil 16e geführt ist. Durch Führungsnuten 23a im Gehäuseteil 16e und Führungszapfen 24 im Kragen 22 entsteht eine Verdrehsicherung, die für die Spindel 14 mit dem Stosskörper 5 nur lineare Bewegungen zulässt. Entsprechend dem zulässigen Hub für das Zusammenpressen von Sack 9 und entsprechend der Gewindesteigung zwischen Mutter 13 und Spindel 14 wird die Drehrichtung des Hohlwellenmotors 10 nach einer bestimmten Anzahl von Umdrehungen umgesteuert.

4

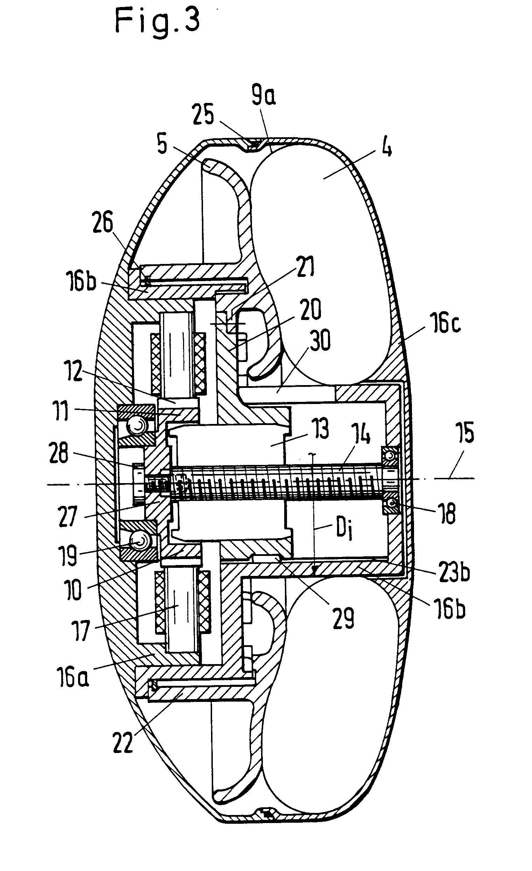

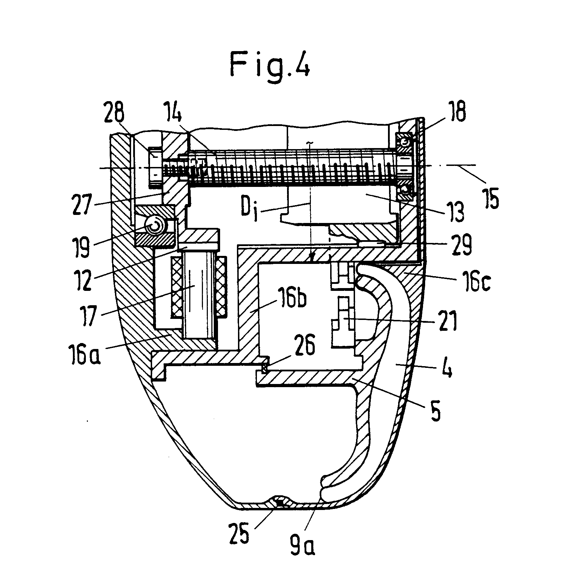

In Fig. 3 und 4 ist unter Beibehaltung der Aussenform mit den Gehäuseteilen 16a, 16c, 25 eine weitere Konfiguration der Innenteile gezeigt. Die Kammer 4 besteht aus einem torus-ähnlichen Sack 9a, wobei die Anschlussleitungen zwischen Kammer 4 und den Rückschlagklappen 2, 3 nicht gezeigt sind. Innerhalb des torus-ähnlichen Sacks 9a mit Innendurchmesser $D_i$ ist eine Spindel 14 gelagert auf der die Mutter 13 periodisch aus- und einfährt. Der elektrische Hohlwellenmotor 10 ist mit seinem Statorteil 17 im Gehäuse 16a befestigt. Der Rotor 12 ist als Hohlwelle 11 ausgeführt, die über einen Teller 27 koaxial durch eine Schraube 28 starr mit der Spindel 14 verbunden ist. Der Rotor 12 ist im Gehäuse 16a über ein Kugellager 19 und innerhalb des torus-ähnlichen Sacks 9a über ein Stützlager 18 abgestützt. Das Stützlager 18 ist in einem Stützkörper 16b eingelassen, der mit dem Gehäuseteil 16a starr verbunden ist. Damit sind Montage und Ueberprüfen der Funktionstüchtigkeit des Antriebs 6 nicht vom Verschliessen des Gehäuses 16 mit Gehäuseteil 16c abhängig, obwohl der Lagerabstand für den Rotor 12 fast der ganzen axialen Länge entspricht. Auf der Spindel 14 reitet die Mutter 13, welche über Speichen 20 mit Pratzen 21 des Stosskörpers 5 verbunden ist. Die Mutter 13 ist über einen eingelassenen Keil 29, der in einer Längsnut 23b des Stützkörpers 16b läuft, gegen Verdrehung gesichert und bewegt sich mit der Drehung der Spindel 14 in den Rotor 12 oder in den torus-ähnlichen Sack 9a hinein. Auf diese Weise wird in Richtung der Längsachse 15 eine kurze axiale Bauweise erreicht, die wegen des Raumgewinns eine Implantation zur Unterstützung des natürlichen Herzens möglich macht. Zur Herabsetzung der Reibung zwischen Mutter 13 und Spindel 14 ist es vorteilhaft, einen Gewinderollenschraubtrieb (roller screw, vis à rouleau) einzusetzen, bei dem die Bewegung zwischen Mutter 13 und Spindel 14 durch Wälzkörper übertragen wird. Gewinderollenschraubtriebe sind z.B. im Lieferprogramm der ROLLVIS S.A., chemin du Pont-du-Centenaire, CH-1228 Genève geführt. Der Stützkörper 16b besitzt Aussparungen 30, durch die die Speichen 20 der Mutter 13 zum ringförmigen Stosskörper 5 herausragen. Zur Reduktion der axialen Bauhöhe tauchen Speichen 20 und Pratzen 21 zeitweise auch axial zwischen den radialen Verbindungen des Stützkörpers 16b ein. Der Stosskörper 5 ist über einen Kragen 22 axial verlängert und stützt sich mit einem Führungsring 26 an einem zylindrischen Teil des Stützkörpers 16b ab. Der Stosskörper 5 ist damit unabhängig von seiner Lage ohne Biegemoment auf Mutter 13 an einer zylindrischen Aussenfläche und einer axial dazu versetzten Innenfläche von Stützkörper 16b geführt, was den Verschleiss zwischen Mutter 13 und Spindel verringert. Wechselbelastungen und Verschleiss sind wesentliche Risikofaktoren für die Funktionstüchtigkeit, wenn man z.B. einen Einsatz von 2 Jahren mit 120 Ausstossbewegungen pro Minute annimmt. Aus diesem Grund ist in den Figuren 2 und 3 die Gewindelänge der Mutter 13 grösser als das 2-Fache des Gewindedurchmessers der Spindel 14. In Fig. 4 ist der torus-ähnliche Sack 9a in zusammengepresstem Zustand gezeigt. Es wäre durchaus denkbar, den Stützkörper von der entgegen-gesetzten Seite auf den Sack 9a wirken zu lassen und ihn mit einer Mutter 13 auf den Antrieb 6 zuzuziehen. Dies bedingt jedoch eine durchgehende Zwischenwand zwischen Sack 9a und elektrischem Hohlwellenmotor 10, an der sich der Sack abstützen kann und entlang welcher die Anschlussleitungen für die Kammer 4 herausgeführt werden müssen.

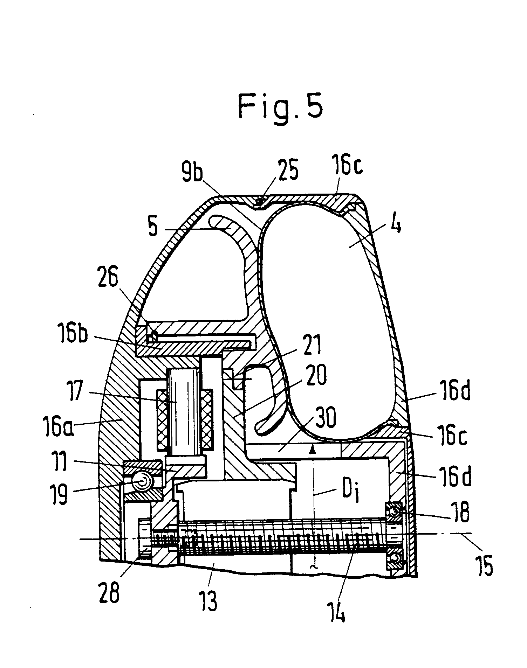

Fig. 5 entspricht der Fig. 3, wobei der torus-ähnliche Sack 9a durch eine Rollmembran 9b ersetzt und dichtend zwischen dem Gehäuse 16c und einem Gehäusering 16d verpresst ist. Die Anschlüsse zu den Rückschlagklappen 2 und 3 liegen im Gehäusering 16d.

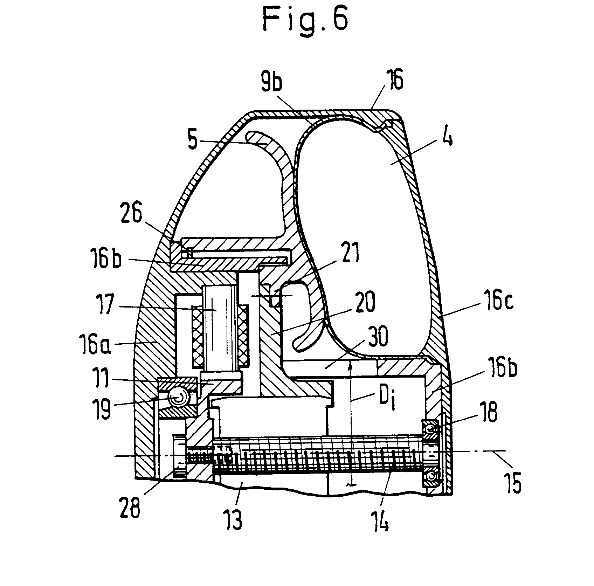

In Fig. 6 ist das Gehäuse 16 aus zwei äusseren Gehäuseteilen 16a und 16c zusammengesetzt, wobei die Einspannung der Membrane 9b gleichzeitig Gehäusedichtung bildet.

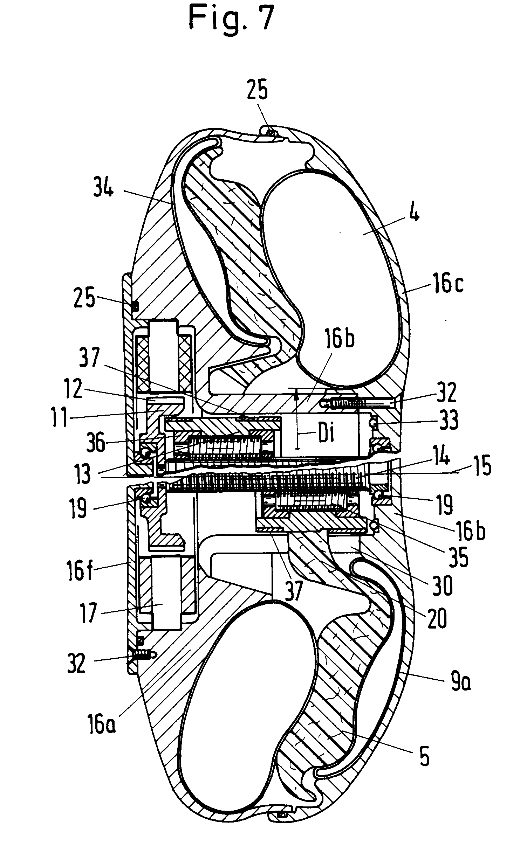

In Fig. 7 ist eine Ausführung mit zwei torus-ähnlichen Säcken 9a, 34 gezeigt, die wechselseitig von dem dazwischen liegenden Stosskörper 5 zusammengepresst werden. Stosskörper 5 und Gehäuseteile 16a, 16c sind so ausgebildet, dass eine Abrollbewegung der torus-ähnlichen Säcke 9a, 34 beim Zusammenpressen und beim Füllen unter Innendruck stattfindet. Die Verdrehsicherung des Stosskörpers 5 und der mit ihm verbundenen Mutter 13 findet zwischen den Speichen 20 und den Aussparungen 30 im Stützkörper 16b statt. Die eigentliche Längsführung des Stosskörpers 5, der auf der Mutter 13 fixiert ist, geschieht durch Führungsringe 37, die ebenfalls auf der Mutter 13 fixiert sind und im Stützkörper 16b laufen. Planetenrollen 35, die in in der Mutter 13 drehbar gelagerten Lageringen 36 geführt sind, rollen mit ihrem Gewinde und unter leichter Vorspannung auf dem Gewinde der Spindel 14 und dem Innengewinde der Mutter 13 ab. Eine Endlagendämpfung 33 wird durch eine Weichdichtung gebildet. Der zweite torus-ähnliche Sack 34, der ohne wesentliche Vergrösserung der Aussenabmessungen des Gehäuses einbaubar ist, eröffnet mehrere Möglichkeiten:

Zum einen können beide torus-ähnlichen Säcke 9a, 34 als Blutsäcke für den gleichen Bypass benutzt werden, wenn jeder Sack individuelle Zufluss- und Ausstossleitungen mit Rückschlagklappen aufweist. Beide Hubrichtungen werden für Bluttransport ausgenutzt und es wird gleichmässiger Förderarbeit abgegeben. Das notwendige Ausgleichsvolumen für das im Gehäuse eingeschlossene Gas wird geringer. Wegen der Nutzung des Hubes in beiden Richtungen kann die Baugrösse reduziert werden.

Zum anderen kann ein Sack als Blutsack und der andere Sack als geschlossener Gasspeicher ausgeführt werden. Auf diese Weise bleibt das notwendige Ausgleichsvolumen für das im Gehäuse eingeschlossene Gas immer noch klein, während der als Gasspeicher verwendete Sack einen Arbeitsspeicher bildet, der seine Arbeit während der Kompression des Blutsacks abgibt. Sofern die Kompression des Gasspeichers nicht zu hoch gewählt ist, wird dem Antriebsmotor weniger Spitzenleistung abverlangt.

Eine weitere Anwendung der beiden torus-ähnlichen Säcke 9a, 34 besteht darin, dass ein Sack zu der linken Herzkammer und der andere Sack zu der rechten Herzkammer einen Bypass mit individuellen Rückschlagklappen 2, 3 bildet.

**Interne stückliste**

| 1 | Pumpelement |
|---|---|
| 2 | Rückschlagklappe oder -ventil |
| 3 | Rückschlagklappe oder -ventil |
| 4 | Kammer |
| 5 | Stosskörper |
| 6 | Antrieb |
| 7 | Zuflussrichtung |
| 8 | Ausstossrichtung |
| 9 | Sack |
| 9a | torus-ähnlicher Sack |
| 9b | Membran |
| 10 | elektrischer Hohlwellenmotor |
| 11 | Hohlwelle |
| 12 | Rotor |
| 13 | Mutter |
| 14 | Spindel |
| 15 | Längsachse (axiale Richtung) |
| 16 | Gehäuse |
| 16a | Gehäuseteil |
| 16b | Stützkörper |
| 16c | Gehäuseteil |
| 16d | Gehäusering |
| 16e | Gehäusezwischenwand |
| 16f | Gehäusedeckel |
| 17 | Statorteil |
| 18 | Stützlager |
| 19 | Lager |
| 20 | Speiche |
| 21 | Pratze |
| 22 | Kragen |
| 23a | Führungsnut |
| 23b | Führungsnut |
| 24 | Führungzapfen |
| 25 | statische Dichtung |
| 26 | Führungsring |
| 27 | Teller |
| 28 | Schraube |
| 29 | Keil |
| 30 | Aussparung |
| 31 | Anschlussöffnung |
| 32 | Befestigungsschraube |
| 33 | Endlagendämpfung |
| 34 | torus-ähnlicher Sack |
| 35 | Planetenrolle |
| 36 | Lagerring |
| 37 | Führungsring |

**Patentansprüche**

1. Intermittiernd fördernde Pumpe zum Fördern von Blut bestehend aus einer Rückschlagklappe (2), durch die Blut zufliesst, und aus einem Pumpelement (1), das Blut gegen einen höheren Druck durch eine Rückschlagklappe (3) ausstösst, wobei das Pumpelement (1) eine deformierbare Kammer (4) und einen Antrieb (6) mit einem auf die Kammer (4) wirkenden Stosskörper (5) aufweist, dadurch **gekennzeichnet**, dass die Kammer (4) aus einem druckfesten deformierbaren Sack (9) besteht, dass der Antrieb (6) aus einem elektrischen Hohlwellenmotor (10) besteht, dass mit der Drehung dessen Hohlwelle (11) ein über Mutter (13) und Spindel (14) gebildeter Linearantrieb auf den Stosskörper (5) einwirkt, und dass Mutter (13) und Spindel (14) innerhalb des als Hohlwelle (11) ausgebildeten Rotors (12) des Elektromotors (10) angeordnet sind.

2. Pumpe nach Anspruch 1, dadurch gekennzeichnet, dass die Kammer (4) als torus-ähnlicher Sack (9a) ausgeführt ist, dessen Innendurchmesser $D_i$ so gross dimensioniert ist, dass Verschiebeelemente (13, 14) des Linearantriebs darin einschiebbar sind.

3. Pumpe nach Anspruch 2, dadurch gekennzeichnet, dass der als Hohlwelle (11) augebildete Rotor (12) einseitig mit der Spindel (14) verbunden ist und dass eine mit dem Stosskörper (5) verbundene Mutter (13), die gegenüber dem Gehäuse (16) verdrehsicher längsgeführt ist, in den Rotor (12) eintaucht.

4. Pumpe nach Anspruch 2 und 3, dadurch gekennzeichnet, dass mit dem Gehäuseteil (16a), das den Statorteil (17) des elektrischen Hohlwellenmotors aufnimmt, ein Stützkörper (16b) verbunden ist, der innerhalb des Innendurchmessers $D_i$ in den torus-ähnlichen Sack (9a) hineinragt und ein Stützlager (18) für den durch die Spindel (14) verlängerten Rotor (12) bildet.

5. Pumpe nach Anspruch 2 und 3, dadurch gekennzeichnet, dass radiale Übertragungselemente (20, 21) zwischen Stosskörper (5) und Mutter (13) sich gegenseitig mit dem Stützkörper (16b) in axialer Richtung (15) durchdringen, um den Stützkörper (16b) innerhalb des Durchmessers $D_i$ vom torus-ähnlichen Sack (9a) durchzuführen.

6. Pumpe nach Anspruch 2, dadurch gekennzeichnet, dass die Kammer (4) von der Antriebsseite weg gegen den gegenüberliegenden Gehäuseteil (16c) durch den Stosskörper (5) zusammenpressbar ist.

7. Pumpe nach Anspruch 1 oder 2, dadurch gekennzeichnet, dass die Gewindelänge der Mutter (13) mindestens eine Länge von zwei Gewindedurchmessern der Spindel (14) aufweist, um den Verschleiss im Gewinde klein zu halten.

8. Pumpelement nach einem der Ansprüche 1 bis 7, dadurch gekennzeichnet, dass der bewegte Teil des deformierbaren Sacks (9, 9a) durch eine zwischen Gehäuse (16c) und Stosskörper (5) dichtende Membran (9b) ersetzt ist und dass die Kammer (4) durch Gehäuse (16d) und Membran (9b) gebildet ist.

9. Pumpe nach einem der Ansprüche 1 bis 8, dadurch gekennzeichnet, dass ein zweiter torus-ähnlicher Sack (34) zwischen Stosskörper (5) und Gehäuseteil (16a) angebracht ist, der ebenfalls vom Stosskörper (5) im Volumen veränderbar ist.

10. Pumpe nach Anspruch 9, dadurch gekennzeichnet, dass einer der beiden Säcke (9a, 34) als Blutsack angeschlossen ist und dass der andere Sack als Speicher mit Gas gefüllt ist.

11. Pumpe nach Anspruch 9, dadurch gekennzeichnet, dass beide Säcke (9a, 34) als Blutsäcke mit individuellen Rückschlagklappen (2, 3) verbunden sind.

12. Pumpe nach einem der Ansprüche 1 bis 11, dadurch gekennzeichnet, dass die Mutter (13) aus einer Hülse besteht in der Lagerringe (36) drehbar gelagert sind, welche Planetenrollen (35) führen, die mit ihrem Gewinde auf dem Gewinde der Spindel (14) und auf dem Innengewinde der Mutter (13) abrollen.

# Fig.1

# Fig.2

# Fig.3

# Fig.4

# Fig.5

# Fig.6

# Fig. 7

EP 0 478 499 A1

**Europäisches Patentamt**

**EUROPÄISCHER RECHERCHENBERICHT**

Nummer der Anmeldung

EP 91 81 0689

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | IEEE TRANSACTIONS ON BIO-MEDICAL ELECTRONICS, Band 37, Nr. 2, Februar 1990, Seiten 138-144, New York, US; W.J. WEISS: "Permanent circulatory support systems at the Pennsylvania State University" * Seite 138, Absatz 2 - Seite 139, Absatz 2; Figur 2 * --- | 1 | A 61 M 1/10 F 04 B 43/08 |
| A | IDEM --- | 9,11,12 | |
| Y | EP-A-0 231 687 (SNAI AEROSPATIALE) * Spalte 9, Zeile 26 - Spalte 10, Zeile 33; Figuren 5,10 * --- | 1-3,6 | |
| Y | US-A-4 004 299 (RUNGE) * Spalte 2, Zeile 29 - Spalte 4, Zeile 44; Figuren 2,3 * --- | 1-3,6 | |
| A | US-A-4 822 357 (FORSTER) * Spalte 2, Zeile 54 - Spalte 3, Zeile 68; Figur 3 * --- | 1,2,6 | RECHERCHIERTE SACHGEBIETE (Int. Cl.5) |
| A | DE-A-3 810 660 (NIPPON ZEON) * Spalte 3, Zeile 10 - Spalte 5, Zeile 13; Figur 1 * --- | 1,7,8 | A 61 M F 04 B |
| A | IEEE ENGINEERING IN MEDICINE AND BIOLOGY MAGAZINE, Band 5, Nr. 1, März 1986, Seiten 30-36, New York, US; K. HAYASHI: "Japanise experience with ventricular assist devices" * Seite 32, Absatz 2 - Seite 33, Absatz 1; Figuren 2,8 * --- -/- | 1,2,7,8 | |

Der vorliegende Recherchenbericht wurde für alle Patentansprüche erstellt

| Recherchenort | Abschlußdatum der Recherche | Prüfer |
|---|---|---|
| DEN HAAG | 11-12-1991 | BERTRAND G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

15

**Europäisches
Patentamt**

# EUROPÄISCHER RECHERCHENBERICHT

Nummer der Anmeldung

EP 91 81 0689

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 825 086 (JÖRN) <br> * Spalte 1, Zeile 52 - Spalte 2, Zeile 15; Figuren 2-4 * <br> --- | 1-3,9, 11,12 | |
| A | ASAIO TRANSACTIONS, Band 33, Nr. 3, September 1987, Seiten 235-239, Hagerstown, MD, US; S. TAKATANI: "Toward a completely implantable TAH" <br> ----- | | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| DEN HAAG | 11-12-1991 | BERTRAND G. |

EPO FORM 1503 03.82 (P0403)